# EUROPÄISCHE PATENTSCHRIFT

(11) **EP 3 698 713 B1**
(45) Veröffentlichungstag und Bekanntmachung des Hinweises auf die Patenterteilung: **06.09.2023**
(21) Anmeldenummer: 20000059.4
(22) Anmeldetag: 10.02.2020
(51) Int. Cl.: A61B 5/053, A61B 5/08, A61B 5/085, A61B 5/00, A61M 16/00, A61B 5/0536, A61B 5/0535, A61B 5/113

(54) **SYSTEM ZUR ERFASSUNG VON ATEMANSTRENGUNGEN EINES PATIENTEN**
SYSTEM FOR DETECTING A PATIENT'S RESPIRATORY EFFORT
SYSTÈME DE DÉTECTION DES EFFORTS RESPIRATOIRES D'UN PATIENT

(30) Priorität: 20.02.2019 DE 102019104337
(43) Veröffentlichungstag der Anmeldung: 26.08.2020
(73) Patentinhaber: Löwenstein Medical Technology S.A., 2557 Luxembourg (LU)
(72) Erfinder: Kremeier, Peter, 76149 Karlsruhe (DE); Pulletz, Sven, 49090 Osnabrück (DE)
(74) Vertreter: Marx, Thomas

(56) Entgegenhaltungen:
- EP-A1- 2 228 009
- CN-A- 106 540 364
- DE-A1-102013 203 177
- US-A1- 2006 278 223
- US-A1- 2011 245 711

## Beschreibung

Diese Erfindung bezieht sich auf Vorrichtung, die geeignet und ausgebildet ist, die Änderung der elektrischen Impedanz eines Körperabschnitts während der Atmung / Beatmung unter Verwendung elektrisch leitfähiger Elektroden zu ermitteln. Diese Erfindung bezieht sich im Allgemeinen auf die elektrische Impedanzanalyse (EI) und beispielsweise auf die elektrische Impedanztomographie (EIT). Ein übliches Verfahren zum Erhalten von Bilddaten mittels EIT ist es, einen elektrischen Strom in ein Paar von leitenden Elektroden einzugeben und Potentiale zu messen, die zwischen einem anderen Paar von leitenden Elektroden erzeugt werden.

EIT wird auf dem Gebiet der medizinischen Abbildung, als eine Alternative zu Computertomographie-abtastung (CT-Abtastung) oder Magnetresonanz-tomographie (MRI), angewendet. Die EIT weist gegenüber den Alternativen die Vorteile der Nicht-Invasivität, fehlender Bestrahlungsschädigungen und einer langfristigen Überwachung auf. Die Bildauflösung ist hingegen geringer als bei den alternativenMethoden. In den letzen Jahren wurde die EIT-Technologie weiterentwickelt mit dem Ziel, die Bildauflösung weiter zu verbessern. Die DE 102012224522 A1 , die WO 2002053029 A1 und die US 5,544,662 offenbaren Verfahren zum Verbessern der Bildauflösung der EIT. Insbesondere wird die EIT-Technologie zur Überwachung und bildlichen Darstellung der Lunge und der Gasverteilung innerhalb der Lunge eines atmenden Lebewesens genutzt.

Eine Anwendung der EIT wird in der DE 10 2013 203 177 A1 offenbart, bei der Anhand einer Impedanzverteilung und einer pixelweisen Auswertung festgestellt wird, wie und ob der positive endexspiratorische Druck (PEEP) angepasst werden sollte.

Die EP 2 228 009 A1 offenbart eine Vorrichtung, mit welcher über eine Impedanzverteilung und der Betrachtung der globalen Veränderung der Impedanz Aussagen über die Lungenrekrutierung und der intertidalen Gasverteilung getroffen werden.

Eine Methode, bei der aus den EIT-Daten während einer Herz-Druck-Massage die Beatmungskurve und die Phase der Druck-Massage getrennt werden unter anderem um ein Beatmungsgerät entsprechend zu steuern, offenbart die CN 106540364 A.

Auch in der Beobachtung von Atmungsparameter, beispielweise um Herzprobleme vorherzusagen bzw. zu bestimmen, findet die EIT Anwendung, wie die US 2011/0245711 A1 beschreibt.

Eine Methode zur Steuerung eines Beatmungsgerätes anhand von Parametern wie der Atemphase kann der US 2006/0278223 A1 entnommen werden. Als vordergründiger Messparameter wird dabei das Flusssignal verwendet. Weitere Beatmungsparameter können beispielsweise auch per EIT ermittelt werden.

Diese bekannten EIT-Technologien benötigen aufwändige Elektrodenanordnungen, Rechnereinheiten und Berechnungsalgorithmen die aus den, mittels der Elektroden gemessenen, Spannungen die Verteilung der elektrischen Impedanz im Thorax unter Verwendung von Algorithmen abschätzen. Zudem werden EIT-Bildgebungsalgorithmen und viel Rechenleistung benötigt, um aus der Verteilung der elektrischen Impedanz einzelne Bilder, und nachfolgend eine Vielzahl an Bildern, zu erzeugen, um den Verlauf während der Atmung bildlich darzustellen.

Die Aufgabe der vorliegenden Offenbarung liegt darin, ein Verfahren und eine Vorrichtung zum schnellen und effektiven Ermitteln der elektrischen Impedanz (EI) zu schaffen und lediglich Änderungen der Impedanz zu erfassen und auszuwerten. Die Erfindung basiert auf der Erkenntnis, dass aus der Änderungen der Impedanz im zeitlichen Verlauf der Atmung / Beatmung bereits viele für die Beatmung relevante Informationen gewonnen werden können. Die Erfindung basiert auch auf der Erkenntnis, dass die Information der Änderungen der Impedanz im zeitlichen Verlauf der Atmung / Beatmung wichtiger ist, als die aufwändige Gewinnung von Bilddaten.

Nachteile der gegenwärtigen EIT-Techniken werden damit überwunden.

Die Erfindung betrifft daher eine Vorrichtung zum schnellen und effektiven Ermitteln der elektrischen Impedanz (EI) im zeitlichen Verlauf der Atmung / Beatmung, ohne daraus eine Bildinformation gewinnen zu müssen.

Die Vorrichtung zur Erfassung des elektrischen Verhaltens eines lebendigen Körpers während der Atmung und/oder Beatmung, umfasst Mittel zur Beatmung und Mittel zur Erfassung der Impedanz des lebendigen Körpers und zumindest eine Steuereinheit, wobei die Erfassung der Impedanz unter Verwendung von zumindest zwei elektrisch leitfähigen Elektroden erfolgt, die das elektrische Verhalten des lebendigen Körpers erfassen, wobei die Mittel zur Erfassung der Impedanz eingerichtet sind, die Änderung der Impedanz des lebendigen Körpers im Zeitverlauf der Atmung und/oder Beatmung erfassen, wobei die Mittel zur Beatmung eingerichtet sind Atemgashübe für die Inspiration und Exspiration im zeitlichen Wechsel vorzugeben dadurch gekennzeichnet, dass die Steuereinheit zumindest zeitweise Informationen der Impedanz und Informationen der Atmung und/oder Beatmung auswertet.

Die Vorrichtung ist auch dadurch gekennzeichnet, dass die Steuereinheit den Verlauf der Impedanz im zeitlichen Verlauf der Atmung und/oder Beatmung anzeigt und/oder aufzeichnet.

Die Vorrichtung ist auch dadurch gekennzeichnet, dass die Steuereinheit die Steigung der (momentanen) Impedanz bestimmt, wobei Steigung positive und negative Werte (Polarität der Steigung) annehmen kann oder gleich Null sein kann.

Die Vorrichtung ist auch dadurch gekennzeichnet, dass die Steuereinheit auch ausgebildet ist, aus der Polarität der Steigung zu bestimmen, ob eine Inspiration oder eine Exspiration vorliegt. Die Steuereinheit ist auch ausgebildet, bei einer Steigung mit einer ersten Polarität, eine Abflachung der Steigung der Impedanz (die gegen Null gehen kann) zu identifizieren und nachfolgend eine Steigung mit einer zweiten Polarität zu identifizieren.

Die Steuereinheit ist auch ausgebildet, den Wechsel der Polarität der Steigung als den Bereich einer endexspiratorischen Impedanz oder einer endinspiratorischen Impedanz zu interpretieren.

Die Steuereinheit ist auch ausgebildet, den Wechsel der Polarität der Steigung als den Bereich eines Triggerzeitpunktes zu interpretieren.

Die Vorrichtung ist auch dadurch gekennzeichnet, dass die Steuereinheit auch ausgebildet ist, eine Abflachung der Steigung der Impedanz zu erfassen, wobei die Steuerung eine Abflachung der Steigung als eine Annäherung auf einen Wert der maximalen oder der minimalen Impedanz interpretiert.

Die Vorrichtung ist auch dadurch gekennzeichnet, dass die Steuereinheit auch ausgebildet ist, nachdem eine Abflachung der Steigung der Impedanz identifiziert wurde, den Übergang zu einem Punkt zu ermitteln, bei dem die Steigung im Wesentlichen Null ist und diesen als einen Wert der maximalen oder der minimalen Impedanz zu interpretieren.

Die Vorrichtung ist auch dadurch gekennzeichnet, dass die Steuereinheit maximale Werte der Impedanz als inspiratorische Impedanz wertet.

Die Vorrichtung ist auch dadurch gekennzeichnet, dass die Steuereinheit minimale Werte der Impedanz als exspiratorische Impedanz wertet.

Die Vorrichtung ist auch dadurch gekennzeichnet, dass für die inspiratorische Impedanz ein Schwellwert festgelegt ist und/oder automatisch ermittelt wird.

Die Vorrichtung ist auch dadurch gekennzeichnet, dass Impedanzen die unterhalb des Schwellwertes liegen als Fehltrigger bewertet werden.

Die Vorrichtung ist auch dadurch gekennzeichnet, dass inspiratorische Impedanzen die unterhalb des Schwellwertes liegen als Fehltrigger bewertet werden.

Die Vorrichtung ist auch dadurch gekennzeichnet, dass Impedanzen, die oberhalb eines zeitlich unmittelbar vorangegangenen Minimalwertes der Impedanz liegen, jedoch unterhalb des Schwellwertes liegen, als Fehltrigger bewertet werden.

Die Vorrichtung ist auch dadurch gekennzeichnet, dass Fehltrigger registriert und gespeichert und/oder an das Beatmungsgerät und/oder den Impedanzmonitor übermittelt werden.

Die Vorrichtung ist auch dadurch gekennzeichnet, dass die Fehltrigger vom Beatmungsgerät und/oder dem Impedanzmonitor im Bereich einer Anzeige unmittelbar visualisiert werden oder abrufbar gespeichert werden.

Die Vorrichtung ist auch dadurch gekennzeichnet, dass die Steuereinheit aus dem Wechsel von zunehmender und abnehmender Impedanz und im Zeitverlauf die Frequenz der Atmung / Beatmung bestimmt.

Die Vorrichtung ist auch dadurch gekennzeichnet, dass die Steuereinheit die endinspiratorische Impedanz als Triggerkriterium für die Steuerung der folgenden Exspiration durch das Beatmungsgerät verwendet.

Die Vorrichtung ist auch dadurch gekennzeichnet, dass die Steuereinheit die endexspiratorische Impedanz als Triggerkriterium für die Steuerung der folgenden Inspiration durch das Beatmungsgerät verwendet.

Die Vorrichtung ist auch dadurch gekennzeichnet, dass die Steuereinheit die Frequenz der Beatmungsvorgabe mit der Eigen-Frequenz des Patienten, bestimmt aus der Impedanz, vergleicht.

Die Vorrichtung ist auch dadurch gekennzeichnet, dass die Steuereinheit die Frequenz der Beatmungsvorgabe mit der Eigen-Frequenz des Patienten, bestimmt aus der Impedanz und/oder dem Fluss oder dem Druck, vergleicht.

Die Vorrichtung ist auch dadurch gekennzeichnet, dass die Steuereinheit den Zeitpunkt der Beatmungsvorgabe mit dem Zeitpunkt des Impedanzsignals, vergleicht.

Die Vorrichtung ist auch dadurch gekennzeichnet, dass die Impedanzinformation von der Steuereinheit genutzt wird, die mechanische Druck- und Volumenbelastung unter Beatmung zu bewerten und die Vorgabe der Beatmung entsprechend adaptiert.

Die Vorrichtung ist auch dadurch gekennzeichnet, dass von der Steuereinheit für zumindest eine Atmung und/oder Beatmung ein erster PEEP-Druck vorgegeben wird und dabei eine erste endexspiratorische Impedanz ermittelt wird und für zumindest eine Atmung und/oder Beatmung ein zweiter PEEP-Druck vorgegeben und dabei eine zweite endexspiratorische Impedanz ermittelt wird und zumindest die erste und die zweite (oder beliebige weitere) endexspiratorische Impedanzen miteinander verglichen werden und für denjenigen PEEP-Druck, der die geringste endexspiratorische Impedanz bewirkt, eine Empfehlung für den Bediener des Beatmungsgerätes gespeichert oder ausgegeben wird oder eine automatische Auswahl und Anwendung dieses PEEP-Druckes erfolgt.

Die Vorrichtung ist auch dadurch gekennzeichnet, dass die Steuereinheit aus zumindest einem maximalen Wert der Impedanz und zumindest einem minimalen Wert der Impedanz eine Impedanz-Variation ermittelt.

Die Vorrichtung ist auch dadurch gekennzeichnet, dass die Impedanz-Variation ermittelt und genutzt wird um zumindest eine Beatmungseinstellung (42), wie beispielsweise Druck, PEEP, die Drucksteuerung in der Exspiration, Frequenz, Volumen und/oder auch die Empfindlichkeit der Sensoren, adaptiv so zu verändern, dass die Impedanz-Variation zunimmt.

Die Vorrichtung ist auch dadurch gekennzeichnet, dass für zumindest eine Atmung und/oder Beatmung eine erste Beatmungseinstellung vorgegeben und dabei eine erste Impedanz-Variation ermittelt wird und anschließend für zumindest eine Atmung und/oder Beatmung eine zweite Beatmungseinstellung vorgegeben und dabei die eine zweite Impedanz-Variation ermittelt wird und zumindest die erste und die zweite (oder beliebige weitere) Impedanz-Variationen miteinander verglichen werden und für diejenige Beatmungseinstellung die die höchste Impedanz-Variation bewirkt, eine Empfehlung für den Bediener des Beatmungsgerätes gespeichert oder ausgegeben wird oder eine automatische Auswahl und Anwendung dieser Beatmungseinstellung erfolgt.

Die Erfindung betrifft auch eine Vorrichtung zur Erfassung des elektrischen Verhaltens eines lebendigen Körpers während der Atmung und/oder Beatmung, umfassend Mittel zur Beatmung und Mittel zur Erfassung der Impedanz des lebendigen Körpers und zumindest eine Steuereinheit, wobei die Erfassung der Impedanz unter Verwendung von zumindest zwei elektrisch leitfähigen Elektroden erfolgt, die das elektrische Verhalten des lebendigen Körpers erfassen, wobei die Mittel zur Erfassung der Impedanz eingerichtet sind, die Änderung der Impedanz des lebendigen Körpers im Zeitverlauf der Atmung und/oder Beatmung erfassen, wobei die Mittel zur Beatmung eingerichtet sind Atemgashübe für die Inspiration und Exspiration vorzugeben dadurch gekennzeichnet, dass die Steuereinheit zumindest zeitweise Informationen der Impedanz und Informationen der Atmung und/oder Beatmung auswertet für zumindest eine Atmung und/oder Beatmung eine erste Beatmungseinstellung vorgegeben und dabei eine erste Impedanz-Variation ermittelt wird und anschließend für zumindest eine Atmung und/oder Beatmung eine zweite Beatmungseinstellung vorgegeben und dabei die eine zweite Impedanz-Variation ermittelt wird und zumindest die erste und die zweite (oder beliebige weitere) Impedanz-Variationen miteinander verglichen werden und für diejenige Beatmungseinstellung die die höchste Impedanz-Variation bewirkt, eine Empfehlung für den Bediener des Beatmungsgerätes gespeichert oder ausgegeben wird oder eine automatische Auswahl und Anwendung dieser Beatmungseinstellung erfolgt.

Die Vorrichtung ist auch dadurch gekennzeichnet, dass die Vorrichtung einteilig aufgebaut ist und Mittel zur Beatmung und Mittel zur Erfassung der Impedanz und die Steuereinheit umfasst.

Die Vorrichtung ist auch dadurch gekennzeichnet, dass die Vorrichtung mehrteilig aufgebaut ist und die Mittel zur Beatmung und die Mittel zur Erfassung der Impedanz und die Steuereinheit räumlich getrennt angeordnet sind und funktional zusammenwirken.

Die Erfindung betrifft auch eine Vorrichtung zur Erfassung des elektrischen Verhaltens eines lebendigen Körpers während der Atmung und/oder Beatmung, umfassend Mittel zur Beatmung und Mittel zur Erfassung der Impedanz des lebendigen Körpers und zumindest eine Steuereinheit, wobei die Erfassung der Impedanz unter Verwendung von zumindest zwei elektrisch leitfähigen Elektroden erfolgt, die das elektrische Verhalten des lebendigen Körpers erfassen, wobei die Mittel zur Erfassung der Impedanz eingerichtet sind, die Änderung der Impedanz des lebendigen Körpers im Zeitverlauf der Atmung und/oder Beatmung erfassen, wobei die Mittel zur Beatmung eingerichtet sind Atemgashübe für die Inspiration und Exspiration vorzugeben dadurch gekennzeichnet, dass die Steuereinheit die Steigung der (momentanen) Impedanz bestimmt, wobei Steigung positive und negative Werte (Polarität der Steigung) annehmen kann oder gleich Null sein kann.

Die Offenbarung betrifft alternativ und/oder ergänzend auch ein Verfahren und eine Vorrichtung zum schnellen und effektiven Ermitteln der elektrischen Impedanz (EI) im zeitlichen Verlauf der Atmung / Beatmung, mit Ermittlung einer Bildinformation der elektrischen Impedanz(-verteilunq).

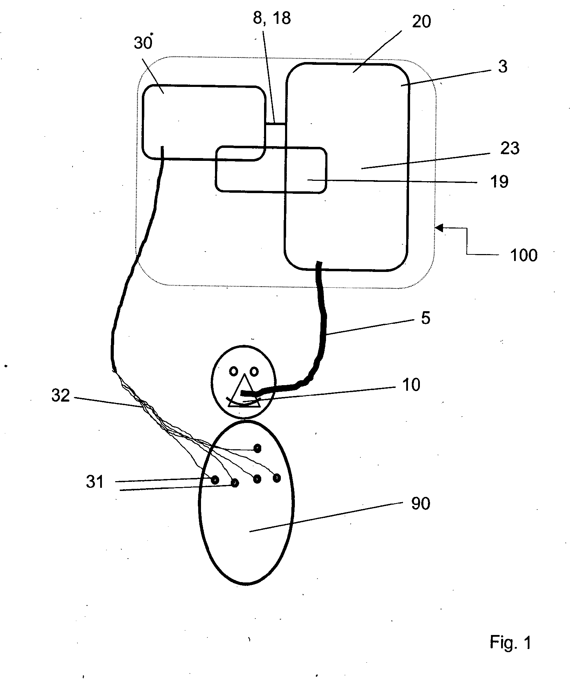

Fig. 1 zeigt schematisch die Vorrichtung (100) mit der Anordnung von Beatmungsgerät (20) und Impedanzmonitor (30) der ein Teil des Beatmungsgerätes sein kann oder ein separates Gerät ist, welches bevorzugt funktional mit dem Beatmungsgerät zusammenwirkt, am Patienten (90). Die Messung der Impedanz (40) basiert auf der Verwendung elektrisch leitfähiger Elektroden (31) die aktiv oder passiv das elektrische Verhalten des menschlichen Körpers (90) erfassen.

Die fortlaufend oder phasenweise gemessene bzw. bestimmte Impedanz (40) erlaubt, die Änderung der Impedanz des Thorax während der Atmung oder unter Beatmung zu bewerten.

Bei dem erfindungsgemäßen Impedanzmonitor (30) wird ein elektrischer Strom an die Haut des Thorax angelegt, um ein elektrisches Feld im Thorax aufzubauen. Erfindungsgemäß sind 2, 4, 8, 16, 32 oder mehr Elektroden (31) um den Thorax herum angeordnet und werden zur Messung, der aus dem Feld resultierenden elektrischen Potentiale, verwendet. Die gemessenen Spannungen werden verwendet, um die Änderung der elektrischen Impedanz im Thorax unter Verwendung von Algorithmen zu ermitteln. Der Verzicht auf eine Ermittlung der Impedanzverteilung und der Verzicht auf eine aufwändige Berechnung von Bilddaten ermöglichen eine sehr schnelle und kostengünstige Nutzung der Impedanzinformation.

Erfindungsgemäß werden Änderungen der Impedanz relativ zu einer Basis- oder Referenzbestimmung ermittelt. Beispielsweise wird so die Änderung der Impedanz im Verlauf der Atmung / Beatmung ermittelt, wobei die Impedanz mit der Inspiration ansteigt und mit der Exspiration fällt. Dieser relative Ansatz eleminiert Fehler, die sich aus Annahmen bezüglich Thoraxform, Elektrodenposition oder Körperzusammensetzung, wie bei der EIT-Technologie üblich, ergeben.

Die Änderungen der Impedanz zeigt somit nicht ihren absoluten Wert an.

Erfindungsgemäß werden begrenzte Strommengen (typisch 0,5 - 100 mA) genutzt, da nur geringe Ströme einen maximalen Signal-Rausch-Abstand erreichen. Die Elektroden sind an diskreten physischen Orten um den Brustkorb oder auf dem Brustkorb verteilt, beispielsweise in äquidistantem Abstand.

Die Elektroden (31) können isolierte Gelelektroden oder EKG-Elektroden sein, die durch einzelne abgeschirmte Kabel mit entfernt angeordneten elektronischen Schaltungen verbunden sind.

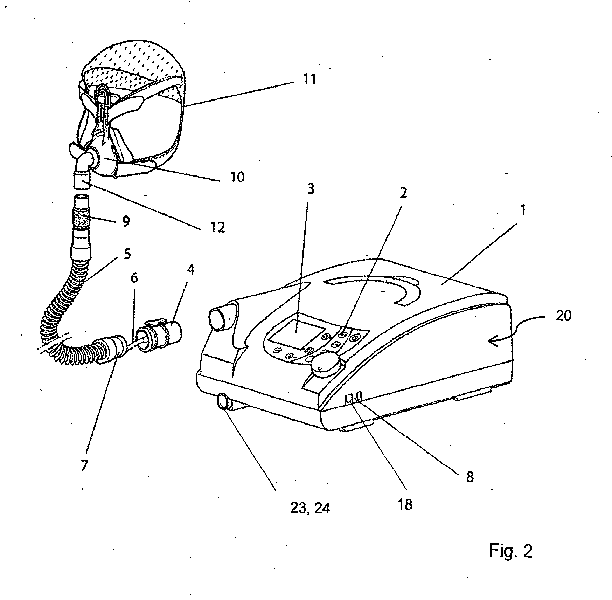

Fig. 2 zeigt den grundsätzlichen Aufbau eines Beatmungsgerätes (20). Im Bereich eines Gerätegehäuses (1) sind ein Bedienelement (2) und/oder ein Bedien-und Informationssystem (3) angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8,18) auf. Ein Anfeuchter (21) oder ein Vernebler (22) kann zudem adaptiert werden. Das Beatmungsgerät weist eine Atemgasquelle (17) auf

Im Bereich einer dem Gerätegehäuse (1) abgewandten Ausdehnung des Verbindungsschlauches (5) ist beispielhaft ein Ausatmungselement (9) angeordnet. Ebenfalls kann ein Ausatemventil verwendet werden. Das Beatmungsgerät (20) kann als Schlaftherapiegerät, als High-Flow-Gerät, als Anästhesiegerät, als klinisches- oder Heim- oder Notfall-Beatmungsgerät ausgebildet sein.

Fig. 2 zeigt darüber hinaus ein als Beatmungsmaske (10) ausgebildetes Patienten-Interface. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf. Das Patienten-Interface kann beispielsweise auch als Tubus oder andere Schnittstelle ausgebildet sein.

Über die Schnittstelle (8,18) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Bevorzugt ist auch ein Kartenschacht vorgesehen. Die Schnittstelle (8) kann auch als LAN-Schnittstelle oder als sonstige Schnittstelle zur Anbindung an das Internet oder an einen Patientenmonitor oder ein EI-Gerät (30) ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Das erfindungsgemäße Beatmungsgerät (20) ist so ausgelegt, dass es über einen Schlauch und ein Patienteninterface mit einem Patienten verbunden werden kann, um eine Beatmung bereitzustellen. Es umfasst eine Quelle für Atemgas (17), die beispielsweise als ein Elektromotor mit Gebläserad oder als Druckgasanschluss mit zumindest einem Ventil ausgebildet ist. Das Beatmungsgerät weist eine Einrichtung zur Ermittlung von Druck und/oder Fluss und/oder Volumen (23, 24) des Atemgases auf. Eine Steuereinheit (19) ist so ausgelegt, dass sie beispielsweise für jeden Atemzyklus auf der Basis eines vorbestimmten Wertes und/oder auf Basis von Messsignalen für die Parameter Druck und/oder Flow und/oder Volumen einen Atemgasparameter bestimmt und die Quelle für Atemgas derart reguliert, dass der Atemgasparameter appliziert wird. Die Steuereinheit kann die Parameter der Beatmung kontrolliert vorgeben und/oder zumindest teilweise assistiert oder adaptiv unter Berücksichtigung von Messignalen.

Die Steuereinheit (19) ist beispielsweise so ausgelegt, dass sie den aktuellen Druck und/oder Flow und/oder das Volumen von Atemgas bestimmt. Der aktuelle Wert kann auf der Anzeige (3) darstellt werden.

Weiterhin vergleicht die Steuereinheit (19) solche ParameterWerte, die von einem Benutzer vorgegeben wurden, beispielsweise obere und untere Druckgrenzen oder eine maximal tolerierbare Anzahl von Apnoen pro Zeiteinheit, oder eine maximal tolerierbare Leckage, mit den aktuellen Werten und generiert eine BenutzerInformation zu Abweichungen von der Vorgabe. Die Benutzerinformation wird bevorzugt über das Bedien- und Informationssystem (3) graphisch visualisiert.

Das Beatmungsgerät (20) weist dazu einen (pneumatischen oder elektronischen oder optischen) Druckmesseingang und einen Drucksensor (23) auf.

Die Steuereinheit (19) ist beispielsweise eingerichtet und ausgebildet eine Veränderung der Impedanz zu identifizieren und daraufhin das Beatmunggerät zur Vorgabe eines Beatmungsparameters anzusteuern.

Bei Überschreiten oder Unterschreiten eines Schwellwertes für die Impedanz generiert die Steuereinheit (19) beispielsweise ein Steuersignal für das Beatmungsgerät (20), zur Vorgabe eines inspiratorischen oder exspiratorischen Atemgasdrucks. Bei Überschreiten oder Unterschreiten eines Schwellwertes für die Impedanz generiert die Steuereinheit (19) beispielsweise alternativ ein Steuersignal für das Beatmungsgerät (20), zur Beendigung der Vorgabe eines inspiratorischen oder exspiratorischen Atemgasdrucks.

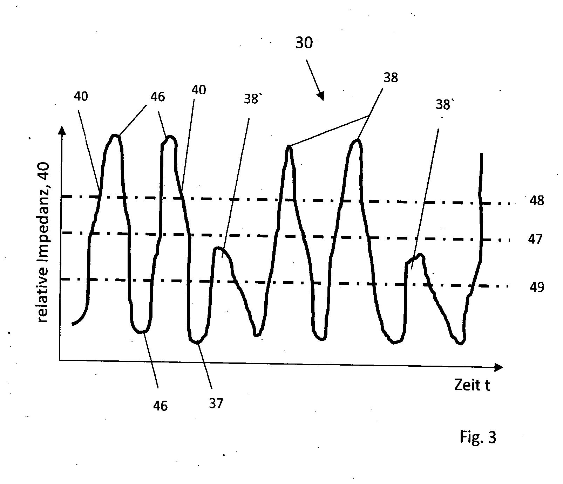

Fig. 3 zeigt schematisch den Verlauf der Impedanz (40) des Thorax während der Atmung. Dargestellt ist der Verlauf des Summensignals der Impedanz, wobei das Summensignal mit der Inspiration (38, 38') ansteigt und der Exspiration abfällt. Die Impedanz ist hier ohne Einheiten als relative Impedanz (40) aufgetragen. Der Impedanzmonitor (30) erfasst die Impedanz (40), beispielsweise des Thorax, mit einer Frequenz von beispielsweise 6 - 200 Messungen pro Sekunde. Die Software erstellt aus den Messwerten die relativen Impedanzen (40).

Die Impedanz wechselt, im Rhythmus der Atmung / Beatmung, zwischen Werten einer maximalen (38) und einer minimalen (37) Impedanz. Die fortlaufende Aufzeichnung der Impedanz erlaubt eine Bestimmung der Steigung (45) der (momentanen) Impedanz zu jedem Zeitpunkt. Die Steigung kann dabei positive und negative Werte (Polarität der Steigung) annehmen. Man erkennt, dass die Steigung (45) bei der Annäherung auf einen Wert der maximalen (38) oder der minimalen (37) Impedanz abnimmt und irgendwann einen Punkt erreicht, bei dem die Steigung im Wesentlichen Null (46) ist und nachfolgend wieder ansteigt.

Die Steuereinheit (19) ist daher bevorzugt ausgebildet, die Steigung (45) der Impedanz (40) zu bestimmen. Die Steuereinheit (19) ist auch ausgebildet, die Polarität der Steigung zu bestimmen. Die Steuereinheit (19) ist daher bevorzugt auch ausgebildet, eine Änderung der Steigung (45) der Impedanz (40) zu erfassen. Die Steuereinheit (19) ist auch ausgebildet, eine Abflachung der Steigung (45) der Impedanz (40) zu erfassen, wobei die Steuerung eine Abflachung der Steigung (45) als eine Annäherung auf einen Wert der maximalen (38) oder der minimalen (37) Impedanz interpretiert. Die Steuereinheit (19) ist auch ausgebildet, nachdem eine Abflachung der Steigung (45) der Impedanz (40) identifiziert wurde, den Übergang zu einem Punkt zu ermitteln, bei dem die Steigung im Wesentlichen Null (46) ist und nachfolgend wieder ansteigt. Die Steuereinheit (19) ist auch ausgebildet, solche Punkte, bei denen die Steigung Wesentlichen Null (46) ist als endexspiratorische Impedanz (37) und/oder als endinspiratorische Impedanz (38) zu interpretieren. Die Steuereinheit (19) ist auch ausgebildet, bei einer Steigung mit einer ersten Polarität, eine Abflachung der Steigung (45) der Impedanz (40) (die gegen Null (46) gehen kann) zu identifizieren und nachfolgend eine Steigung mit einer zweiten Polarität zu identifizieren. Die Steuereinheit (19) ist auch ausgebildet, den Wechsel der Polarität der Steigung als den Bereich einer endexspiratorischen Impedanz (37) oder einer endinspiratorischen Impedanz (38) zu interpretieren. Die Steuereinheit (19) ist auch ausgebildet, den Wechsel der Polarität der Steigung als den Bereich eines Triggerzeitpunktes zu interpretieren. Die Steuereinheit (19) ist auch ausgebildet, aus der Polarität der Steigung zu bestimmen, ob eine Inspiration oder eine Exspiration vorliegt.

Für die weitere Auswertung wird beispielsweise die endexspiratorische Impedanz (37) und/oder die endinspiratorische Impedanz (38) betrachtet. Die endinspiratorische Impedanz (38) ist die Impedanz am Ende einer Einatmung. Für die inspiratorische Impedanz (38) kann ein Schwellwert (48) festgelegt werden und/oder automatisch ermittelt werden. Inspiratorische Impedanzen (38) die unterhalb des Schwellwertes (48) liegen werden als Fehltrigger (38') bewertet. Hier führt eine Einatembemühung des Patienten nicht zu einer Inspiration (durch das Beatmungsgerät).

Analog dazu kann auch ein Schwellwert (49) für die minimale, exspiratorische Impedanz (37) definiert und genutzt werden.

Der Schwellwert kann auch die durchschnittliche Impedanz (47) sein. Erfindungsgemäß werden die Fehltrigger (38') registriert und gespeichert und/oder an das Beatmungsgerät (20) und/oder den Impedanzmonitor (30) übermittelt. Die Fehltrigger (38') können vom Beatmungsgerät (20) und/oder dem Impedanzmonitor (30) im Bereich einer Anzeige (3) unmittelbar visualisiert werden oder abrufbar gespeichert werden.

Erfindungsgemäß können die Fehltrigger (38') als eine Anzahl der Fehltrigger (38') pro Zeiteinheit gespeichert und ausgegeben werden. Der Schwellwert (48, 49) ist erfindungsgemäß voreingestellt und/oder kann durch den Nutzer festgelegt werden und/oder ist zumindest teilweise autoadaptiv, in Abhängigkeit von zumindest der maximalen Impedanz, einstellbar. Der Schwellwert (48, 49) kann bei einem Prozentwert der maximalen Impedanz (38) liegen, beispielsweise im Bereich 66% bis 33% der maximalen Impedanz (38) oder im Bereich unter 75% der maximalen Impedanz (38) oder im Bereich unter 50% der maximalen Impedanz. Der Schwellwert (48, 49) kann auch in Abhängigkeit von der minimalen Impedanz (37) festgelegt werden, beispielsweise muss die minimalen Impedanz (37) um zumindest 40% überschritten sein, oder um zumindest 70% überschritten sein, um auf keine Fehltriggerung (38') zu schließen.

Erfindungsgemäß ist auch daran gedacht die Fehltrigger (38') zu nutzen, um die Beatmungseinstellungen, wie beispielsweise Druck, Frequenz, Volumen und auch die Empfindlichkeit der Sensoren, adaptiv so zu verändern, dass die Anzahl der Fehltrigger (38') abnimmt.

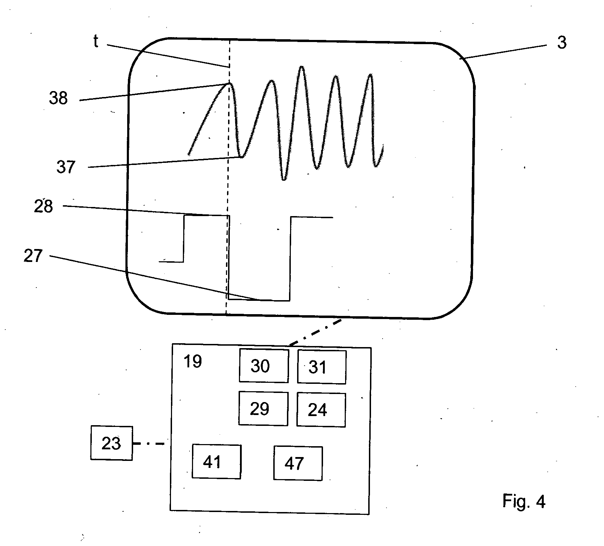

Fig. 4 zeigt oben schematisch den Verlauf der Impedanz (40) des Thorax während der Atmung im Bereich einer Anzeige (3). Für die weitere Auswertung wird beispielsweise die endexspiratorische Impedanz (37), die endinspiratorische Impedanz (38) dargestellt.

Zudem zeigt die Anzeige (3) unten den Verlauf von Vorgaben des Beatmungsgerätes oder Messwerte der Sensoren die hier dem Druckverlauf entsprechen mit inspiratorischen Drücken (28) und exspiratorischen Drücken (27). Eingeblendet ist ebenfalls eine Zeitpunktinformation (t) die die Kurven der Impedanz und der Beatmungsvorgabe visuell verbindet. Im unteren Teil von Fig. 4 ist insbesondere die Steuereinheit (19) zu erkennen, die die notwendigen Messwerte und Informationen für die Darstellung des Verlaufes der Änderung der Impedanz (40) und der Beatmung / Atmung sammelt und aufbereitet.

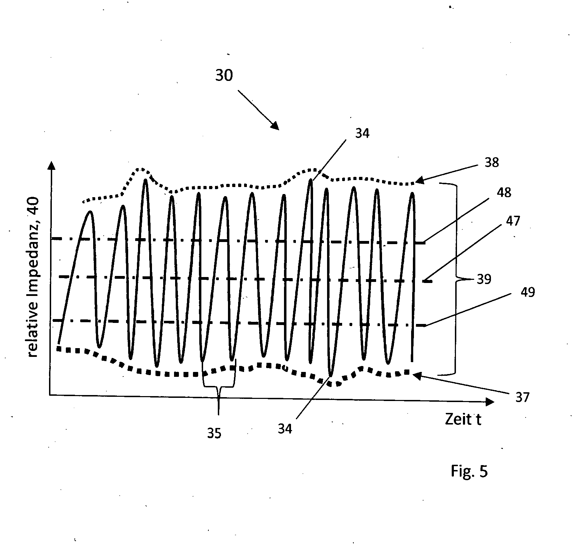

Fig. 5 zeigt schematisch den Verlauf der Impedanz (40) des Thorax während der Atmung. Die Impedanz ist hier ohne Einheiten als relative Impedanz (40) aufgetragen. Der Impedanzmonitor (30) erfasst die Impedanz (40), beispielsweise des Thorax, mit einer Frequenz von beispielsweise 60 (oder im Bereich 20 - 100) Messungen pro Sekunde. Die Software erstellt aus den Messwerten die relativen Impedanzen (40) und gibt sie beispielsweise als Zahlenwerte wieder. Für die weitere Auswertung wird beispielsweise die endexspiratorische Impedanz (37), die endinspiratorische Impedanz (38) und die Impedanz-Variation (39) genutzt.

Die Impedanz-Variation (39) ist die Differenz aus der endexspiratorischen (niedrigsten) und der endinspiratorischen (höchsten) Impedanz; sie repräsentiert die Änderung der Thoraximpedanz innerhalb eines Atemzuges. Eine Zunahme der Impedanz-Variation (39) deutet auf eine Zunahme des Atemzugvolumens hin. Grob vereinfacht ist die Belüftung der Lunge dann verbessert.

Die endexspiratorische Impedanz (37) ist die Impedanz am Ende einer Ausatmung. Die endinspiratorische Impedanz (38) ist die Impedanz am Ende einer Einatmung.

Erfindungsgemäß ist auch daran gedacht endexspiratorische Impedanz (37) zu nutzen, um die Beatmungseinstellungen, wie beispielsweise Druck, PEEP, die Drucksteuerung in der Exspiration, Frequenz, Volumen und/oder auch die Empfindlichkeit der Sensoren, adaptiv so zu verändern, dass die endexspiratorische Impedanz (37) abnimmt.

Erfindungsgemäß ist auch daran gedacht endexspiratorische Impedanz (37) für die Einstellung des optimalen PEEP zu nutzen. Dafür wird für zumindest einen Atemzug oder für mehrere Atemzüge ein erster PEEP-Druck vorgegeben und dabei eine erste endexspiratorische Impedanz (37) ermittelt. Dann wird für zumindest einen Atemzug oder für mehrere Atemzüge ein zweiter PEEP-Druck vorgegeben und dabei die eine zweite endexspiratorische Impedanz (37) ermittelt. Erfindungsgemäß können in diesem Sinne eine Vielzahl von PEEP-Drücke vorgegeben werden und eine Vielzahl von entsprechenden endexspiratorischen Impedanzen (37) ermittelt werden. Zumindest die erste und die zweite (oder beliebige weitere) endexspiratorische Impedanzen (37) werden dann miteinander verglichen. Für denjenigen PEEP-Druck (41best), der die geringste endexspiratorische Impedanz (37best) bewirkt, wird eine Empfehlung für den Bediener des Beatmungsgerätes gespeichert oder ausgegeben oder es erfolgt eine automatische Auswahl und Anwendung dieses PEEP-Druckes (41best).

Dafür werden für zumindest einen Atemzug oder für mehrere Atemzüge systematisch unterschiedliche PEEP-Drücke vorgegeben

Erfindungsgemäß ist auch daran gedacht endinspiratorische Impedanz (38) zu nutzen, um die Beatmungseinstellungen, wie beispielsweise Druck, PEEP, die Drucksteuerung in der Inspiration, Frequenz, Volumen und/oder auch die Empfindlichkeit der Sensoren, adaptiv so zu verändern, dass die endinspiratorische Impedanz (38) zunimmt.

Aus dem Wechsel von endexspiratorischer Impedanz (37) und endinspiratorischer Impedanz (38) im Zeitverlauf kann der Impedanzmonitor die Frequenz (35) der Atmung / Beatmung bestimmen.

Die höchste, endinspiratorische Impedanz (38) kann als Triggerkriterium (34) für die Steuerung der folgenden Exspiration durch das Beatmungsgerät (20) verwendet werden.

Die niedrigste, endexspiratorische Impedanz (37) kann als Triggerkriterium (34) für die Steuerung der folgenden Inspiration durch das Beatmungsgerät (20) verwendet werden.

Die Impedanz-Variation (39) spiegelt die Ventilationsänderungen in der Lunge wider. Erfindungsgemäß kann eine Betrachtung der gesamten Lunge, als auch eine getrennte Auswertung für ventralen und dorsale oder beliebige Lungenabschnitte erfolgen.

Eine Zunahme der Impedanz-Variation (39) deutet auf eine Zunahme des Atemzugvolumens hin.

Erfindungsgemäß ist auch daran gedacht die Impedanz-Variation (39) zu nutzen, um die Beatmungseinstellungen, wie beispielsweise Druck, PEEP, die Drucksteuerung in der Exspiration, Frequenz, Volumen und/oder auch die Empfindlichkeit der Sensoren, adaptiv so zu verändern, dass die Impedanz-Variation (39) zunimmt.

Dafür wird für zumindest einen Atemzug oder für mehrere Atemzüge ein erster PEEP-Druck vorgegeben und dabei eine erste Impedanz-Variation (39) ermittelt. Dann wird für zumindest einen Atemzug oder für mehrere Atemzüge ein zweiter PEEP-Druck vorgegeben und dabei die eine zweite Impedanz-Variation (39) ermittelt. Erfindungsgemäß können in diesem Sinne eine Vielzahl von PEEP-Drücke vorgegeben werden und eine Vielzahl von entsprechenden Impedanz-Variation (39) ermittelt werden. Zumindest die erste und die zweite (oder beliebige weitere) Impedanz-Variation (39) werden dann miteinander verglichen. Für denjenigen PEEP-Druck (41c), der die höchste Impedanz-Variation (39c) bewirkt, wird eine Empfehlung für den Bediener des Beatmungsgerätes gespeichert oder ausgegeben oder es erfolgt eine automatische Auswahl und Anwendung dieses PEEP-Druckes (41c).

Erfindungsgemäß ist auch daran gedacht die Impedanz-Variation (39) zu nutzen, um zumindest eine Beatmungseinstellung (42), wie beispielsweise Druck, PEEP, die Drucksteuerung in der Exspiration, Frequenz, Volumen und/oder auch die Empfindlichkeit der Sensoren, adaptiv so zu verändern, dass die Impedanz-Variation (39) zunimmt. Dafür wird für zumindest einen Atemzug oder für mehrere Atemzüge eine erste Beatmungseinstellung (42) vorgegeben und dabei eine erste Impedanz-Variation (39) ermittelt. Dann wird für zumindest einen Atemzug oder für mehrere Atemzüge eine zweite Beatmungseinstellung (42) vorgegeben und dabei die eine zweite Impedanz-Variation (39) ermittelt. Erfindungsgemäß können in diesem Sinne eine Vielzahl von Beatmungseinstellungen (42) vorgegeben werden und eine Vielzahl von entsprechenden Impedanz-Variationen (39) ermittelt werden. Zumindest die erste und die zweite (oder beliebige weitere) Impedanz-Variationen (39) werden dann miteinander verglichen. Für diejenige Beatmungseinstellung (42a) die die höchste Impedanz-Variation (39a) bewirkt, wird eine Empfehlung für den Bediener des Beatmungsgerätes gespeichert oder ausgegeben oder es erfolgt eine automatische Auswahl und Anwendung dieser Beatmungseinstellung (42a).

## Patentansprüche

1. Vorrichtung (100) zur Erfassung des elektrischen Verhaltens (40) eines lebendigen Körpers (90) während der Atmung und/oder Beatmung, umfassend Mittel zur Beatmung (20) und Mittel zur Erfassung (30) der Impedanz des lebendigen Körpers (90) und zumindest eine Steuereinheit (19), wobei die Erfassung der Impedanz (40) unter Verwendung von zumindest zwei elektrisch leitfähigen Elektroden (31) erfolgt, die das elektrische Verhalten des lebendigen Körpers (90) erfassen, wobei die Mittel zur Erfassung der Impedanz (30) eingerichtet sind, die Änderung der Impedanz des lebendigen Körpers (90) im Zeitverlauf der Atmung und/oder Beatmung erfassen, wobei die Mittel zur Beatmung (20) eingerichtet sind Atemgashübe für die Inspiration (28) und Exspiration (27) im zeitlichen Wechsel vorzugeben, wobei die Steuereinheit (19) zumindest zeitweise Informationen der Impedanz (40) und Informationen der Atmung und/oder Beatmung (25, 26, 27, 28) auswertet, **dadurch gekennzeichnet, dass** für die inspiratorische und/oder exspiratorische Impedanz ein Schwellwert (47, 48, 49) festgelegt ist und/oder automatisch ermittelt wird und Impedanzen (40) die unterhalb oder oberhalb des Schwellwertes (47, 48, 49) liegen als Fehltrigger (38') bewertet werden.

2. Vorrichtung nach Anspruch 1 **dadurch gekennzeichnet, dass** die Steuereinheit (19) den Verlauf der Impedanz (40) im zeitlichen Verlauf der Atmung und/oder Beatmung (25, 26, 27, 28) anzeigt und/oder aufzeichnet.

3. Vorrichtung nach zumindest einem der vorhergehenden Ansprüche **dadurch gekennzeichnet, dass** die Steuereinheit die Steigung (45) der (momentanen) Impedanz (40) bestimmt, wobei Steigung positive und negative Werte (Polarität der Steigung) annehmen kann oder gleich Null (46) sein kann, wobei die Steuereinheit (19) auch ausgebildet ist, aus der Polarität der Steigung zu bestimmen, ob eine Inspiration oder eine Exspiration vorliegt.

4. Vorrichtung nach Anspruch 3 **dadurch gekennzeichnet, dass** die Steuereinheit (19) auch ausgebildet ist, eine Abflachung der Steigung (45) der Impedanz (40) zu erfassen, wobei die Steuerung eine Abflachung der Steigung (45) als eine Annäherung auf einen Wert der maximalen (38) oder der minimalen (37) Impedanz interpretiert.

5. Vorrichtung nach zumindest einem der vorhergehenden Ansprüche 3 und 4 **dadurch gekennzeichnet, dass** die Steuereinheit (19) auch ausgebildet ist, nachdem eine Abflachung der Steigung (45) der Impedanz (40) identifiziert wurde, den Übergang zu einem Punkt zu ermitteln, bei dem die Steigung im Wesentlichen Null (46) ist und diesen als einen Wert der maximalen (38) oder der minimalen (37) Impedanz zu interpretieren.

6. Vorrichtung nach zumindest einem der vorhergehenden Ansprüche 4 und 5 **dadurch gekennzeichnet, dass** die Steuereinheit (19) maximale Werte der Impedanz (40) als inspiratorische Impedanz (38) wertet und minimale Werte der Impedanz (40) als exspiratorische Impedanz (37) wertet.

7. Vorrichtung nach zumindest einem der vorhergehenden Ansprüche 4 bis 6 **dadurch gekennzeichnet, dass** Impedanzen (40), die oberhalb eines zeitlich unmittelbar vorangegangenen Minimalwertes der Impedanz (37) liegen, jedoch unterhalb des Schwellwertes (48, 49) liegen, als Fehltrigger (38') bewertet werden.

8. Vorrichtung nach zumindest einem der vorhergehenden Ansprüche **dadurch gekennzeichnet, dass** Fehltrigger (38') registriert und gespeichert und/oder an das Beatmungsgerät (20) und/oder den Impedanzmonitor (30) übermittelt werden und dass die Fehltrigger (38') vom Beatmungsgerät (20) und/oder dem Impedanzmonitor (30) im Bereich einer Anzeige (3) unmittelbar visualisiert werden oder abrufbar gespeichert werden.

9. Vorrichtung nach zumindest einem der vorhergehenden Ansprüche **dadurch gekennzeichnet, dass** die Steuereinheit (19) aus dem Wechsel von zunehmender und abnehmender Impedanz im Zeitverlauf eine Frequenz (35) der Atmung / Beatmung bestimmt.

10. Vorrichtung nach zumindest einem der vorhergehenden Ansprüche 4 bis 9 **dadurch gekennzeichnet, dass** die Steuereinheit (19) die endinspiratorische Impedanz (38) als Triggerkriterium (34) für die Steuerung der folgenden Exspiration durch das Beatmungsgerät (20) verwendet und die endexspiratorische Impedanz (37) als Triggerkriterium (34) für die Steuerung der folgenden Inspiration durch das Beatmungsgerät (20) verwendet.

11. Vorrichtung nach zumindest einem der vorhergehenden Ansprüche 9 bis 10 **dadurch gekennzeichnet, dass** die Steuereinheit (19) die Frequenz der Beatmungsvorgabe (25) mit der Eigen-Frequenz (35) des Patienten, bestimmt aus der Impedanz (37, 38, 40), vergleicht.

12. Vorrichtung nach zumindest einem der vorhergehenden Ansprüche 9 bis 11 **dadurch gekennzeichnet, dass** die Steuereinheit (19) den Zeitpunkt der Beatmungsvorgabe (25, 27, 28) mit dem Zeitpunkt des Impedanzsignals (35, 37, 38, 40), vergleicht.

13. Vorrichtung nach zumindest einem der vorhergehenden Ansprüche **dadurch gekennzeichnet, dass** die Impedanzinformation (34-39) von der Steuereinheit genutzt wird, die mechanische Druck- und Volumenbelastung unter Beatmung zu bewerten und die Vorgabe der Beatmung entsprechend adaptiert.

14. Vorrichtung nach zumindest einem der vorhergehenden Ansprüche **dadurch gekennzeichnet, dass** von der Steuereinheit (19) für zumindest eine Atmung und/oder Beatmung ein erster PEEP-Druck vorgegeben wird und dabei eine erste endexspiratorische Impedanz (37) ermittelt wird und für zumindest eine Atmung und/oder Beatmung ein zweiter PEEP-Druck vorgegeben und dabei eine zweite endexspiratorische Impedanz (37) ermittelt wird und zumindest die erste und die zweite (oder beliebige weitere) endexspiratorische Impedanzen (37) miteinander verglichen werden und für denjenigen PEEP-Druck (41b), der die geringste endexspiratorische Impedanz (37b) bewirkt, eine Empfehlung für den Bediener des Beatmungsgerätes gespeichert oder ausgegeben wird oder eine automatische Auswahl und Anwendung dieses PEEP-Druckes (41b) erfolgt.

15. Vorrichtung nach zumindest einem der vorhergehenden Ansprüche 4 bis 14 **dadurch gekennzeichnet, dass** die Steuereinheit (19) aus zumindest einem maximalen Wert der Impedanz und zumindest einem minimalen Wert der Impedanz eine Impedanz-Variation (39) ermittelt.

16. Vorrichtung nach Anspruch 15 **dadurch gekennzeichnet, dass** die Impedanz-Variation (39) ermittelt und genutzt wird um zumindest eine Beatmungseinstellung (42), wie beispielsweise Druck, PEEP, die Drucksteuerung in der Exspiration, Frequenz, Volumen und/oder auch die Empfindlichkeit der Sensoren, adaptiv so zu verändern, dass die Impedanz-Variation (39) zunimmt.

17. Vorrichtung nach zumindest einem der vorhergehenden Ansprüche 15 und 16 **dadurch gekennzeichnet, dass** die Steuereinheit (19) dazu eingerichtet ist, für zumindest eine Atmung und/oder Beatmung eine erste Beatmungseinstellung (42) vorzugegeben und dabei eine erste Impedanz-Variation (39) zu ermittelnund anschließend für zumindest eine Atmung und/oder Beatmung eine zweite Beatmungseinstellung (42) vorzugegeben und dabei die eine zweite Impedanz-Variation (39) zu ermitteln und zumindest die erste und die zweite (oder beliebige weitere) Impedanz-Variationen (39) miteinander verglichen werden und die Steuereinheit (19) ist weiter dazu eingerichtet für diejenige Beatmungseinstellung (42a) die die höchste Impedanz-Variation (39a) bewirkt, eine Empfehlung für den Bediener des Beatmungsgerätes zu speichern oder auszugegeben oder eine automatische Auswahl und Anwendung dieser Beatmungseinstellung (42a) erfolgen zu lassen.

## Claims

1. An apparatus (100) for detecting the electrical behavior (40) of a living body (90) during respiration and/or ventilation, comprising means for ventilation (20) and means for detecting (30) the impedance of the living body (90) and at least one control unit (19), wherein the detection of the impedance (40) takes place using at least two electrically conductive electrodes (31) which detect the electrical behavior of the living body (90), wherein the means for detecting the impedance (30) are configured to detect the change in the impedance of the living body (90) over time during respiration and/or ventilation, wherein the means for ventilation (20) are configured to stipulate respiratory gas volumes for inspiration (28) and expiration (27) alternately in time, wherein the control unit (19) at least intermittently evaluates information relating to the impedance (40) and information relating to the respiration and/or ventilation (25, 26, 27, 28), **characterized in that** a threshold value (47, 48, 49) is defined and/or automatically ascertained for the inspiratory and/or expiratory impedance and impedances (40) that are above or below the threshold value (47, 48, 49) are assessed as failed triggers (38').

2. The apparatus according to claim 1, **characterized in that** the control unit (19) displays and/or records the curve of the impedance (40) over time during respiration and/or ventilation (25, 26, 27, 28).

3. The apparatus according to at least one of the preceding claims, **characterized in that** the control unit determines the slope (45) of the (current) impedance (40), wherein the slope can adopt positive and negative values (polarity of the slope) or be equal to zero (46), wherein the control unit (19) is also designed to determine whether inspiration or expiration is present from the polarity of the slope.

4. The apparatus according to claim 3, **characterized in that** the control unit (19) is also designed to detect a flattening of the slope (45) of the impedance (40), wherein the controller interprets a flatness of the slope (45) as an approach to a value of the maximum (38) or minimum (37) impedance.

5. The apparatus according to at least one of the preceding claims 3 and 4, **characterized in that** the control unit (19) is also designed, after a flattening of the slope (45) of the impedance (40) has been identified, to ascertain the transition to a point at which the slope is substantially zero (46) and to interpret this point as a value of the maximum (38) or minimum (37) impedance.

6. The apparatus according to at least one of the preceding claims 4 and 5, **characterized in that** the control unit (19) classifies maximum values of the impedance (40) as inspiratory impedance (38) and minimum values of the impedance (40) as expiratory impedance (37).

7. The apparatus according to at least one of the preceding claims 4 to 6, **characterized in that** impedances (40) that are above a temporally immediately preceding minimum value of the impedance (37) but that are below the threshold value (48, 49) are assessed as failed triggers (38').

8. The apparatus according to at least one of the preceding claims, **characterized in that** failed triggers (38`) are registered and stored and/or transmitted to the ventilator (20) and/or impedance monitor (30) and **in that** the failed triggers (38`) are directly visualized in the region of a display (3) or stored in a recallable manner by the ventilator (20) and/or impedance monitor (30).

9. The apparatus according to at least one of the preceding claims, **characterized in that** the control unit (19) determines a frequency (35) of the respiration/ventilation from a change of increasing and decreasing impedance over time.

10. The apparatus according to at least one of the preceding claims 4 to 9, **characterized in that** the control unit (19) uses the end-inspiratory impedance (38) as a trigger criterion (34) for controlling the subsequent expiration by the ventilator (20) and the end-expiratory impedance (37) is used as a trigger criterion (34) for controlling the subsequent inspiration by the ventilator (20).

11. The apparatus according to at least one of the preceding claims 9 to 10, **characterized in that** the control unit (19) compares the frequency of the ventilation target (25) with the inherent frequency (35) of the patient, which is determined from the impedance (37, 38, 40).

12. The apparatus according to at least one of the preceding claims 9 to 11, **characterized in that** the control unit (19) compares the time of the ventilation target (25, 27, 28) with the time of the impedance signal (35, 37, 38, 40).

13. The apparatus according to at least one of the preceding claims, **characterized in that** the impedance information (34-39) is used by the control unit to assess the mechanical pressure and volume load under ventilation and accordingly adapts the ventilation target.

14. The apparatus according to at least one of the preceding claims, **characterized in that** a first PEEP pressure is stipulated for at least one instance of respiration and/or ventilation by the control unit (19) and, in the process, a first end-expiratory impedance (37) is ascertained and a second PEEP pressure is stipulated for at least one instance of respiration and/or ventilation and, in the process, a second end-expiratory impedance (37) is ascertained, and at least the first and the second (or any additional) end-expiratory impedances (37) are compared with one another and a recommendation for the PEEP pressure (41b) that causes the lowest end-expiratory impedance (37b) is stored or output for the operator of the ventilator or an automatic selection and application of this PEEP pressure (41b) takes place.

15. The apparatus according to at least one of the preceding claims 4 to 14, **characterized in that** the control unit (19) ascertains an impedance variation (39) from at least one maximum value of the impedance and at least one minimum value of the impedance.

16. The apparatus according to claim 15, **characterized in that** the impedance variation (39) is ascertained and used to adaptively alter at least one ventilation setting (42), for example pressure, PEEP, the pressure control in expiration, frequency, volume and/or the sensitivity of the sensors, in such a way that the impedance variation (39) increases.

17. The apparatus according to at least one of the preceding claims 15 and 16, **characterized in that** the control unit (19) is configured to stipulate a first ventilation setting (42) for at least one instance of respiration and/or ventilation and, in the process, to ascertain a first impedance variation (39) and, subsequently, to stipulate a second ventilation setting (42) for at least one instance of respiration and/or ventilation and, in the process, to ascertain the a second impedance variation (39) and at least the first and the second (or any additional) impedance variations (39) are compared with one another and the control unit (19) is further configured, for the ventilation setting (42a) that causes the highest impedance variation (39a), to store or output a recommendation for the operator of the ventilator or allows an automatic selection and application of this ventilation setting (42a) to take place.

## Revendications

1. Dispositif (100) pour la détection du comportement électrique (40) d'un corps vivant (90) pendant la respiration et/ou la ventilation, comprenant des moyens pour la respiration (20) et des moyens pour la détection (30) de l'impédance du corps vivant (90), et au moins une unité de commande (19), la détection de l'impédance (40) étant effectuée en utilisant au moins deux électrodes électriquement conductrices (31), lesquelles détectent le comportement électrique du corps vivant (90), les moyens pour la détection de l'impédance (30) étant agencés afin de détecter le changement de l'impédance du corps vivant (90) pendant le déroulement de la respiration et/ou de la ventilation, les moyens pour la ventilation (20) étant agencés afin de spécifier des pulsations de gaz respiratoire pour l'inspiration (28) et l'expiration (27) en alternance temporelle, l'unité de commande (19) évaluant au moins temporairement des informations de l'impédance (40) et des informations de la respiration et/ou de la ventilation (25, 26, 27, 28), **caractérisé en ce qu'**une valeur seuil (47, 48, 49) est définie et/ou déterminée automatiquement pour l'impédance inspiratoire et/ou expiratoire, et des impédances (40) inférieures ou supérieures à la valeur seuil (47, 48, 49) étant considérées comme des déclenchements erronés (38').

2. Dispositif selon la revendication 1, **caractérisé en ce que** l'unité de commande (19) affiche et/ou enregistre l'évolution de l'impédance (40) dans le temps de la respiration et/ou de la ventilation (25, 26, 27, 28).

3. Dispositif selon au moins l'une quelconque des revendications précédentes, **caractérisé en ce que** l'unité de commande détermine la pente (45) de l'impédance (40) (momentanée), la pente pouvant prendre des valeurs positives et négatives (polarité de la pente) ou pouvant être égale à zéro (46), l'unité de commande (19) étant aussi conçue pour déterminer à partir de la polarité de la pente si on est en présence d'une inspiration ou d'une expiration.

4. Dispositif selon la revendication 3, **caractérisé en ce que** l'unité de commande (19) est aussi conçue pour détecter un aplatissement de la pente (45) de l'impédance (40), la commande interprétant un aplatissement de la pente (45) comme un rapprochement d'une valeur de l'impédance maximum (38) ou minimum (37).

5. Dispositif selon au moins l'une des revendications 3 et 4 précédentes, **caractérisé en ce que** l'unité de commande (19) est formée pour déterminer le passage à un point auquel la pente est essentiellement égale à zéro (46) et pour interpréter celui-ci comme une valeur de l'impédance maximum (38) ou minimum (37), après qu'un aplatissement de la pente (45) de l'impédance (40) a été identifié.

6. Dispositif selon au moins l'une quelconque des revendications 4 et 5 précédentes, **caractérisé en ce que** l'unité de commande (19) évalue des valeurs maximum de l'impédance (40) comme impédance inspiratoire (38) et des valeurs minimum de l'impédance (40) comme impédance expiratoire (37).

7. Dispositif selon au moins l'une quelconque des revendications 4 à 6 précédentes, **caractérisé en ce que** des impédances (40) qui sont supérieures à une valeur minimum de l'impédance (37) immédiatement précédente dans le temps, toutefois inférieures à la valeur seuil (48, 49), sont considérées comme des déclenchements erronés (38').

8. Dispositif selon au moins l'une quelconque des revendications précédentes, **caractérisé en ce que** des déclenchements erronés (38') sont détectés et enregistrés et/ou transmis à l'appareil respiratoire (20) et/ou au moniteur d'impédance (30), et **en ce que** les déclenchements erronés (38') peuvent être directement visualisés par l'appareil respiratoire (20) et/ou par le moniteur d'impédance (30) dans la zone d'un affichage (3), ou sont enregistrés de façon consultable.

9. Dispositif selon au moins l'une quelconque des revendications précédentes, **caractérisé en ce que** l'unité de commande (19) détermine une fréquence (35) de la respiration/de la ventilation à partir de l'alternance entre impédance croissante et décroissante dans le temps.

10. Dispositif selon au moins l'une quelconque des revendications 4 à 9 précédentes, **caractérisé en ce que** l'unité de commande (19) utilise l'impédance en fin d'inspiration (38) comme critère de déclenchement (34) pour la commande de l'expiration suivante par l'appareil respiratoire (20) et utilise l'impédance en fin d'expiration (37) comme critère de déclenchement (34) pour la commande de l'inspiration suivante par l'appareil respiratoire (20).

11. Dispositif selon au moins l'une quelconque des revendications 9 à 10 précédentes, **caractérisé en ce que** l'unité de commande (19) compare la fréquence de la spécification de ventilation (25) avec la fréquence propre (35) du patient, déterminée à partir de l'impédance (37, 38, 40).

12. Dispositif selon au moins l'une quelconque des revendications 9 à 11 précédentes, **caractérisé en ce que** l'unité de commande (19) compare l'instant de la spécification de ventilation (25, 27, 28) avec l'instant du signal d'impédance (35, 37, 38, 40).

13. Dispositif selon au moins l'une quelconque des revendications précédentes, **caractérisé en ce que** les informations d'impédance (34-39) sont utilisées par l'unité de commande pour évaluer la charge mécanique de volume et de compression sous ventilation, et pour adapter la spécification de la ventilation de façon corrélative.

14. Dispositif selon au moins l'une quelconque des revendications précédentes, **caractérisé en ce qu'**une première pression PEEP est spécifiée par l'unité de commande (19) pour au moins une respiration et/ou une ventilation, et **en ce qu'**une première impédance en fin d'expiration (37) est alors déterminée, et **en ce qu'**une deuxième pression PEEP est spécifiée pour au moins une respiration et/ou une ventilation, et **en ce qu'**une deuxième impédance en fin d'expiration (37) est alors déterminée, et **en ce qu'**au moins la première et la deuxième (ou de quelconques autres) impédances en fin d'expiration (37) sont comparées entre elles, et **en ce qu'**une recommandation pour l'utilisateur de l'appareil respiratoire est enregistrée ou sortie pour la pression PEEP (41b) qui entraîne l'impédance en fin d'expiration la plus faible (37b), ou **en ce qu'**une sélection et utilisation automatiques de cette pression PEEP (41b) sont effectuées.

15. Dispositif selon au moins l'une quelconque des revendications 4 à 14 précédentes, **caractérisé en ce que** l'unité de commande (19) détermine une variation d'impédance (39) à partir d'au moins une valeur maximum de l'impédance et d'au moins une valeur minimum de l'impédance.

16. Dispositif selon la revendication 15, **caractérisé en ce que** la variation d'impédance (39) est déterminée et utilisée afin de modifier de façon adaptative au moins un réglage de ventilation (42) tel que, par exemple, la pression, la PEEP, la commande de pression dans l'expiration, la fréquence, le volume et/ou également la sensibilité des capteurs, de telle sorte que la variation d'impédance (39) augmente.

17. Dispositif selon au moins l'une quelconque des revendications 15 et 16 précédentes, **caractérisé en ce que** l'unité de commande (19) est agencée afin de spécifier un premier réglage de ventilation (42) pour au moins une respiration et/ou une ventilation, et afin de déterminer alors une première variation d'impédance (39), et afin de spécifier ensuite un deuxième réglage de ventilation (42) pour au moins une respiration et/ou une ventilation, et afin de déterminer alors une deuxième variation d'impédance (39), et **en ce qu'**au moins la première et la deuxième (ou de quelconques autres) variations d'impédance (39) sont comparées entre elles, et **en ce que** l'unité de commande (19) est en outre agencée afin d'enregistrer ou de sortir une recommandation pour l'utilisateur de l'appareil respiratoire pour le réglage de ventilation (42a) qui entraîne la variation d'impédance (39a) la plus élevée, ou afin de faire effectuer une sélection et utilisation automatiques de ce réglage de ventilation (42a).
